(19) Europäisches Patentamt
European Patent Office
Office européen des brevets

(11) Veröffentlichungsnummer: **0 202 493**
**B1**

(12) **EUROPÄISCHE PATENTSCHRIFT**

(45) Veröffentlichungstag der Patentschrift: **24.08.88**

(51) Int. Cl.⁴: **C 07 C 143/70**

(21) Anmeldenummer: **86105599.4**

(22) Anmeldetag: **23.04.86**

(54) **Verfahren zur Herstellung von 5-Fluortoluol-2,4-disulfochlorid.**

(30) Priorität: **03.05.85 DE 3515870**

(43) Veröffentlichungstag der Anmeldung: **26.11.86 Patentblatt 86/48**

(45) Bekanntmachung des Hinweises auf die Patenterteilung: **24.08.88 Patentblatt 88/34**

(84) Benannte Vertragsstaaten: **BE CH DE FR GB IT LI**

(56) Entgegenhaltungen:
**JOURNAL OF PHARMACY AND PHARMACOLOGY, Band 12, 1960, Seiten 419-425, London, GB; B.G. BOGGIANO et al.: "Studies in the field of diuretic drugs"**
**JOURNAL OF ORGANIC CHEMISTRY, Band 27, März 1962, Seiten 951-956, Easton, US; C.W. WHITEHEAD et al.: "Diuretics. VI. 1,2,4-Benzothiadiazine 1,1-dioxides substituted at 2,3,4- and 7-N-sulfamoyl positions"**

(73) Patentinhaber: **BAYER AG, Konzernverwaltung RP Patentabteilung, D-5090 Leverkusen 1 Bayerwerk (DE)**

(72) Erfinder: **Diehl, Herbert, Dr., Walter-Flex-Strasse 17, D-5090 Leverkusen 1 (DE)**
Erfinder: **Käsbauer, Josef, Dr., Morgengraben 1, D-5000 Köln 80 (DE)**
Erfinder: **Wedemeyer, Karlfried, Dr., Bilharzstrasse 7, D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**EP 0 202 493 B1**

Beschreibung

Die Erfindung betrifft ein Verfahren zur Herstellung von 5-Fluortoluol-2,4-disulfochlorid durch Chlorsulfonierung von 3-Fluortoluol.

Aus J. Pharm. Pharmacol. 12, 419 (1960) und J. Org. Chem. 27, 951 (1962) ist bekannt, 3-Fluortoluol mit Chlorsulfonsäure umzusetzen. Nachteilig bei diesem Verfahren sind die ungenügenden Ausbeuten von ca. 50% der Theorie und der eingesetzte grosse Überschuss an Chlorsulfonsäure.

Es wurde nun ein Verfahren zur Herstellung von 5-Fluortoluol-2,4-disulfochlorid durch Chlorsulfonierung von 3-Fluortoluol gefunden, das dadurch gekennzeichnet ist, dass man zunächst das 3-Fluortoluol mit Chlorsulfonsäure und anschliessend mit einem anorganischen Säurechlorid bei Temperaturen von bis zu 130°C bis zum Ende der Gasentwicklung umsetzt, dann das Reaktionsgemisch auf Temperaturen bis zu 200°C erhitzt und nach Beendigung der Gasentwicklung bei tieferer Temperatur mit weiterem anorganischen Säurechlorid umsetzt oder das 3-Fluortoluol mit starken Sulfonierungsmitteln bei erhöhter Temperatur gegebenenfalls in Gegenwart von inerten Lösungs- und/oder Verdünnungsmitteln zur entsprechenden Disulfosäure umsetzt und anschliessend die Disulfosäure mit anorganischen Säurechloriden behandelt.

Als anorganische Säurechloride kommen für das erfindungsgemässe Verfahren in Frage: Thionylchlorid, Phosphortrichlorid, Phosphoroxychlorid, Phosphorpentachlorid und/oder Phosgen, bevorzugt Thionylchlorid.

Als starke Sulfonierungsmittel seien genannt: Oleum, $SO_3$, $SO_3$-Addukte, wie Pyridin-$SO_3$, und/oder Schwefelsäure, bevorzugt $SO_3$.

Die erfindungsgemässe Umsetzung des 3-Fluortoluols mit Chlorsulfonsäure und anschliessend mit einem anorganischen Säurechlorid wird vorteilhafterweise bei Temperaturen von 70 bis 130°C, bevorzugt bei Temperaturen von 90 bis 100°C durchgeführt. Dabei wird im wesentlichen das entsprechende Monosulfochlorid gebildet. Nach Beendigung der Gasentwicklung wird das Reaktionsgemisch vorteilhafterweise auf Temperaturen von 130 bis 200°C, bevorzugt auf Temperaturen von 150 bis 170°C erhitzt. Nach Beendigung der Gasentwicklung wird das Reaktionsgemisch abgekühlt, im allgemeinen auf Temperaturen von etwa 50 bis 130°C, bevorzugt 70 bis 100°C, und mit weiterem anorganischen Säurechlorid umgesetzt (Variante 1).

Nach einer anderen Verfahrensvariante (Variante 2) wird das 3-Fluortoluol mit starken Sulfonierungsmitteln bei Temperaturen im Bereich von etwa 50 bis 120°C, bevorzugt 90 bis 110°C, gegebenenfalls in Gegenwart von inerten Lösungs- und/oder Verdünnungsmitteln zunächst zur Disulfonsäure und anschliessend mit anorganischen Säurechloriden zum entsprechenden Disulfonchlorid umgesetzt.

Als inerte Lösungs- und/oder Verdünnungsmittel kommen dabei vor allem in Frage: Schwefelsäure und Chlorsulfonsäure, bevorzugt Chlorsulfonsäure.

Die inerten Lösungs- und/oder Verdünnungsmittel können sowohl einzeln als auch im Gemisch untereinander eingesetzt werden. Die Menge der eingesetzten Lösungs- und/oder Verdünnungsmittel ist nicht kritisch und kann in weiten Bereichen schwanken. Üblicherweise wird eine Menge von etwa 20 bis 200, bevorzugt 30 bis 100 Gew.-%, bezogen auf 3-Fluortoluol, eingesetzt.

Bei der erfindungsgemässen Umsetzung wird die Chlorsulfonsäure im allgemeinen in einer Menge von etwa 2 bis 5 Mol, bevorzugt 2 bis 2,5 Mol, pro Mol 3-Fluortoluol eingesetzt.

Die Menge an anorganischem Säurechlorid beträgt üblicherweise ca. 2 bis 4 Mol, bevorzugt 2 bis 3 Mol, pro Mol 3-Fluortoluol. Dabei wird bei der Variante 1 zunächst die Umsetzung mit 0,5 bis 2 Mol, bevorzugt 1 bis 1,5 Mol, an anorganischem Säurechlorid pro Mol 3-Fluortoluol durchgeführt und dann nach Beendigung der Gasentwicklung das Reaktionsgemisch mit weiterem anorganischen Säurechlorid (ca. 0,5 bis 2 Mol, bevorzugt 1 bis 1,5 Mol, pro Mol 3-Fluortoluol) umgesetzt.

Die Menge der erfindungsgemäss eingesetzten starken Sulfonierungsmittel beträgt im allgemeinen etwa 2 bis 4 Mol, bevorzugt 2 bis 2,5 Mol, pro Mol 3-Fluortoluol.

Nach Beendigung der erfindungsgemässen Umsetzung kann man zur Isolierung des 5-Fluortoluol--2,4-disulfochlorids überschüssiges Thionylchlorid und Chlorsulfonsäure abdestillieren. Eine weitere Möglichkeit zur Isolierung des Reaktionsprodukts ist das Eingiessen der Reaktionsmischung in Eiswasser und Absaugen des ausgefallenen Produkts. Zur Reinigung kann das Rohprodukt destilliert oder aus einem geeigneten Lösungsmittel, z.B. Ligroin, umkristallisiert werden.

Nach dem erfindungsgemässen Verfahren fällt das 5-Fluortoluol-2,4-disulfochlorid jedoch in so hoher Reinheit an, dass es ohne weitere Reinigung direkt weiter verarbeitet werden kann.

Der Vorteil des erfindungsgemässen Verfahrens gegenüber dem Stand der Technik liegt darin, dass in einer Ausbeute bis zu ca.98% der Theorie ein reines (bis zu 99,8%), isomerenfreies 5-Fluortoluol-2,4-disulfochlorid erhalten wird und durch den geringen Sulfonierungsmittel-Überschuss kein unnötiges Abluft- und Abwasserproblem durch die hydrolytische Aufarbeitung von überschüssigen Sulfonierungsmitteln entsteht bzw. ein technisch aufwendiges Abdestillieren grosser Mengen an Sulfonierungsmitteln vermieden wird.

Das erhaltene 5-Fluortoluol-2,4-disulfochlorid kann in bekannter Weise zu 2,4-Dichlor-5-fluorbenzotrichlorid chloriert werden (vgl. DE-PS 234 913; Frdl. 10, 117) und durch partielle Hydrolyse unter $FeCl_3$-Katalyse zu 2,4-Dichlor-5-fluorbenzoylchlorid weiter umgesetzt werden (vgl. Ullmanns Encyclopädie der technischen Chemie, Band 8, Seite 373, 4. Auflage, 1974).

Das 2,4-Dichlor-5-fluorbenzoylchlorid stellt ein wertvolles Zwischenprdukt zur Herstellung antibakterieller Arzneimittel, insbesondere von 1-Cyclopropyl-6-fluor-1,4-dihydro-7-(1-piperazinyl)-4-oxo-chinolincarbonsäure der nachfolgenden Formel dar (siehe DE-OS 3 033 157):

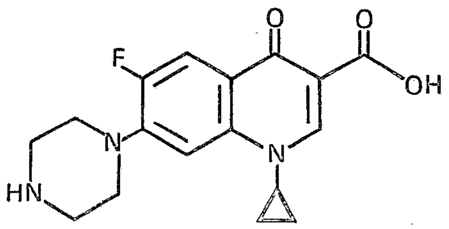

*Beispiele*

*Beispiel 1*

Zu 876 g (7,25 Mol, 0,7% Überschuss) Chlorsulfonsäure (Gehalt 96-97%) tropft man in 65 Minuten 396 g (3,60 Mol) 3-Fluortoluol. Man rührt eine Stunde bei 97-99°C, lässt auf 89°C abkühlen und tropft 588 g (4,94 Mol) Thionylchlorid in 75 Minuten zu. Man rührt 30 Minuten bei 90-95°C nach und heizt in 75 Minuten auf 160°C hoch. Nach dem Ende der Gasentwicklung tropft man bei 78°C 402 g (3,38 Mol) Thionylchlorid in einer Stunde zu. Bei 120°C rührt man bis zum Ende der Gasentwicklung. Überschüssiges Thionylchlorid und etwas Chlorsulfonsäure werden abdestilliert. Beim Abkühlen kristallisiert das Produkt aus. Ausbeute 1087 g, 98,3% der Theorie, Reinheit 99,8%.

*Beispiel 2*

Zu 146 g (1,824 Mol) $SO_3$ tropft man bei 50 bis 80°C in 60 Minuten 99 g (0,899 Mol) 3-Fluortoluol. Man rührt eine Stunde bei 106°C nach, gibt 25 ml Chlorsulfonsäure zu und tropft bei 80-90°C 248 g (2,084 Mol) Thionylchlorid zu. Bei 120°C rührt man bis zum Ende der Gasentwicklung. Die Aufarbeitung erfolgt wie in Beispiel 1. Ausbeute 268,5 g, 97,2% der Theorie, Reinheit 98,7%.

**Patentansprüche**

1. Verfahren zur Herstellung von 5-Fluortoluol-2,4-disulfochlorid durch Chlorsulfonierung von 3-Fluortoluol, dadurch gekennzeichnet, dass man zunächst das 3-Fluortoluol mit Chlorsulfonsäure und anschliessend mit einem organischen Säurechlorid bei Temperaturen bis zu 130°C bis zum Ende der Gasentwicklung umsetzt, dann das Reaktionsgemisch auf Temperaturen von bis zu 200°C erhitzt und nach Beendigung der Gasentwicklung bei tieferer Temperatur mit weiterem anorganischen Säurechlorid umsetzt oder das 3-Fluortoluol mit starken Sulfonierungsmitteln bei erhöhter Temperatur gegebenenfalls in Gegenwart von inerten Lösungs- und/oder Verdünnungsmitteln zur entsprechenden Disulfosäure umsetzt und anschliessend die Disulfosäure mit anorganischen Säurechloriden behandelt.

2. Verfahren nach Anspruch 1, dadurch gekennzeichnet, dass man das 3-Fluortoluol mit Chlorsulfonsäure und anschliessend mit einem anorganischen Säurechlorid bei Temperaturen von 90 bis 100°C umsetzt.

3. Verfahren nach Ansprüchen 1 und 2, dadurch gekennzeichnet, dass man dann das Reaktionsgemisch auf Temperaturen von 150 bis 170°C erhitzt.

4. Verfahren nach Ansprüchen 1 bis 3, dadurch gekennzeichnet, dass man als organische Säurechloride Thionylchlorid, Phosphortrichlorid, Phosphoroxychlorid, Phosphorpentachlorid und/oder Phosgen einsetzt.

5. Verfahren nach Ansprüchen 1 bis 4, dadurch gekennzeichnet, dass man die Chlorsulfonsäure in einer Menge von 2 bis 5 Mol pro Mol 3-Fluortoluol einsetzt.

6. Verfahren nach Ansprüchen 1 bis 5, dadurch gekennzeichnet, dass man pro Mol 3-Fluortoluol 2 bis 4 Mol an anorganischem Säurechlorid einsetzt.

7. Verfahren nach Ansprüchen 1 und 6, dadurch gekennzeichnet, dass man pro Mol 3-Fluortoluol 2 bis 4 Mol an starken Sulfonierungsmitteln einsetzt.

8. Verfahren nach Ansprüchen 1, 6 und 7, dadurch gekennzeichnet, dass man als starke Sulfonierungsmittel Oleum, Schwefeltrioxid, Schwefeltrioxid-Addukte und/oder Schwefelsäure einsetzt.

**Claims**

1. Process for the preparation of 5-fluorotoluene-2,4-disulphochloride by chlorosulphonation of 3-fluorotoluene, characterized in that, first, the 3-fluorotoluene is reacted with chlorosulphonic acid and subsequently with an inorganic acid chloride at temperatures of up to 130°C until the evolution of gas is complete, the reaction mixture is then heated at temperatures of up to 200°C and, after the gas evolution is complete, the product is reacted at a lower temperature with further inorganic acid chloride, or the 3-fluorotoluene is reacted with powerful sulphonating agents at an elevated temperature, if appropriate in the presence of inert solvents and/or diluents, to give the corresponding disulphonic acid, and the disulphonic acid is subsequently treated with inorganic acid chlorides.

2. Process according to Claim 1, characterized in that the 3-fluorotoluene is reacted with chlorosulphonic acid and subsequently with an organic acid chloride at temperatures from 90 to 100°C.

3. Process according to Claims 1 and 2, characterized in that the reaction mixture is then heated at temperatures of 150 to 170°C.

4. Process according to Claims 1 to 3, characterized in that the inorganic acid chlorides employed are thionyl chloride, phosphorus trichloride, phosphorus oxychloride, phosphorus pentachloride and/or phosgene.

5. Process according to Claims 1 to 4, characterized in that the chlorosulphonic acid is employed in an amount of 2 to 5 moles per mole of 3-fluorotoluene.

6. Process according to Claims 1 to 5, characterized in that 2 to 4 moles of inorganic acid chloride are employed per mole of 3-fluorotoluene.

7. Process according to Claims 1 and 6, characterized in that 2 to 4 moles of powerful sulphonating agents are employed per mole of 3-fluorotoluene.

8. Process according to Claims 1, 6 and 7, characterized in that oleum, sulphur trioxide, sulphur trioxide adducts and/or sulphur acid are employed as powerful sulphonating agents.

## Revendications

1. Procédé pour la production de 2,4-disulfochlorure de 5-fluorotoluène par chlorosulfonation du 3-fluorotoluène, caractérisé en ce que l'on transforme tout d'abord le 3-fluorotoluène par l'acide chlorosulfonique et, ensuite, par un chlorure acide inorganique à des températures pouvant atteindre 130°C jusqu'à la fin du dégagement gazeux, puis que l'on chauffe le mélange réactionnel à des températures pouvant atteindre 200°C et qu'à la fin du dégagement gazeux on le transforme à température plus basse avec une quantité supplémentaire de chlorure acide inorganique ou que l'on transforme le 3-fluorotoluène avec des agents de sulfonation puissants à température élevée, éventuellement en présence de solvants et/ou de diluants inertes pour obtenir l'acide disulfonique correspondant et que l'on traite ensuite l'acide disulfonique par des chlorures acides inorganiques.

2. Procédé selon la revendication 1, caractérisé en ce que l'on transforme le 3-fluorotoluène par l'acide chlorosulfonique et, ensuite, par un chlorure acide inorganique à des températures de 90 à 100°C.

3. Procédé selon les revendications 1 et 2, caractérisé en ce que l'on chauffe le mélange réactionnel à des températures de 150 à 170°C.

4. Procédé selon les revendications 1 à 3, caractérisé en ce que l'on utilise comme chlorure acide inorganique le chlorure de thionyle, le trichlorure de phosphore, l'oxychlorure de phosphore, le pentachlorure de phosphore et/ou le phosgène.

5. Procédé selon les revendications 1 à 4, caractérisé en ce que l'on utilise l'acide chlorosulfonique en quantités comprises entre 2 et 5 moles par mole de 3-fluorotoluène.

6. Procédé selon les revendications 1 à 5, caractérisé en ce que l'on utilise 2 à 4 moles de chlorure acide inorganique par mole de 3-fluorotoluène.

7. Procédé selon les revendications 1 à 6, caractérisé en ce que l'on utilise 2 à 4 moles d'agents de sulfonation puissants par mole de 3-fluorotoluène.

8. Procédé selon les revendications 1, 6 et 7, caractérisé en ce que l'on utilise comme agents de sulfonation puissants l'oléum, le trioxyde de soufre, les produits d'addition du trioxyde de soufre et/ou l'acide sulfurique.